# EUROPEAN PATENT SPECIFICATION

(11) **EP 2 844 315 B1**
(45) Date of publication and mention of the grant of the patent: **08.06.2016**
(21) Application number: 13737851.9
(22) Date of filing: 30.04.2013
(51) Int. Cl.: A61M 3/02, A61H 35/04, B05B 11/00, B05B 11/04

(54) **KIT FOR NASAL IRRIGATION**
KIT ZUR NASENSPÜLUNG
COFFRET POUR UNE IRRIGATION NASALE

(30) Priority: 30.04.2012 IT BO20120047 U
(43) Date of publication of application: 11.03.2015
(73) Proprietor: Santini, Daniela Barbara, 37121 Verona (IT)
(72) Inventor: BONER, Tommaso, I-37121 Verona (IT)
(74) Representative: Sandri, Sandro
(86) International application number: PCT/IB2013/053399
(87) International publication number: WO 2013/164759

(56) References cited:
- GB-A- 2 472 327
- US-A- 4 513 891
- US-A1- 2006 253 087
- US-A1- 2011 166 523
- US-B1- 6 736 792

## Description

### Technical field

The present invention relates to a kit for performing nasal irrigation by spraying or dropping a fluid into a nostril.

In particular the present invention relates to a bimodal nasal irrigation kit where nasal irrigation is performed by squeezing a container containing an irrigation fluid or by applying the fluid as drops from the container.

### Background art

At the present time nasal irrigation involves the use of two different types of device. The known devices of the background art include a first device for performing nasal irrigation by squeezing a container containing an irrigation fluid and a second device for performing nasal irrigation whereby a fluid in a container is applied as drops.

These devices have a series of drawbacks

The first drawback is the fact that if users want to perform a squeezable irrigation or perform irrigation with drops they must purchase two items of equipment.

Document US 4513891 A discloses a spray dispenser of the squeeze bottle type, for use in the administration of nasal sprays, comprises a squeeze bottle in combination with a spray dispenser head assembly consisting of a cap, and associated spray nozzle which functions as a two-way check valve and a dip tube for conveying liquid from the bottle to the spray nozzle. The head may optionally be fitted with an overcap for protecting the spray head from contamination when not in use.

Document US 2011166523 A1 discloses a nasal irrigation device includes a container for storing nasal cleansing fluid, a spout having a connecting end, a nose engaging end and a spout passage, the connecting end of the spout being removably coupled to the container to allow the spout passage to receive nasal cleansing fluid from an opening of the container, the spout having a curved portion for directing nasal cleansing fluid toward a nasal passageway of a user when the container is clear of a nasal discharge path, the spout passage being continually open and unobstructed. The container is partially collapsible to force nasal cleansing fluid through the spout passage and into the nasal passageway.

Document US 2006253087 A1 discloses a device and method for treating maladies, particularly of the external ear canal and eardrum, is disclosed. In a preferred embodiment the device comprises an earplug or plug and a delivery bulb that holds treatment fluid. A lumen extends through the earplug and connects the delivery bulb to an orifice located at the distal end of the earplug. A one-way valve is located along the lumen to allow treatment fluid to only flow from the delivery bulb to the orifice and not vice versa. The earplug forms a seal with the patient's external ear canal and, combined with the one-way valve, retains treatment fluid in the patient's external ear canal where it can perform its therapeutic function. In one embodiment, the invention includes a collection bag connected through the earplug to an orifice near the distal end of the earplug to collected waste treatment fluid. The therapeutic method comprises using a device, as disclosed herein, to deliver treatment fluids to the patient's external ear canal for irrigation, for the therapeutic benefit of the treatment fluids for the short and long term or to deliver treatment fluid to a patient's external ear canal where it can pass into the patient's middle ear through a tympanostomy tube placed in the patient's eardrum.

Document US 6736792 B1 discloses a nasal-nasopharyngeal-cleaning (NNC) system is disclosed for removing harmful substances from a human's nasal and nasopharyngeal cavities. Harmful substances herein include infectious agents, chemicals, dust, small particles and dirt deposits in nasal cavities and in the nasopharynx. The NNC system includes a NNC solution, a solution container, a liquid transfer tube, a valve means and a flat-head nostril fitting. The two-step cleaning process comprises cleaning the nasal cavity first with the NNC system and then cleaning the nasopharyngeal cavity with the NNC system. The NNC system is also used to prevent snore.

Document GB 2472327 A discloses a unit dosage of a dry powder formulation suitable for intranasal administration comprises granisteron, where when administered as prophylaxis to cancer patients prior to chemotherapy, a median Cmaxof at least 4 ng/ml is observed. Preferably, the formulation comprises (a) about 1% to 40% of granisetron; (b) about 50% to about 90% of a first crystalline cellulose with a mean particle diameter of 30[mu]m or less; (c) about 5% to about 15% of second crystalline cellulose with a mean particle diameter of 100[mu]m or less; and (d) about 0.1% to about 5% of a fluidizing agent. A single use device may be provided, which comprises a nozzle 6 for positioning into a patient's nostril; a reservoir 23 that includes a single unit dose of powdered therapeutic formulation; a valve assembly 5 coupled to the reservoir; and an air source linked to an upstream end of the valve assembly.

### Disclosure of the invention

The purpose of the present invention is therefore to overcome the drawbacks mentioned above.

This is achieved by means of a nasal irrigation kit having the characteristics described in claim 1. The dependent claims describe particularly advantageous embodiments of the kit according to this invention.

According to the present invention the nasal irrigation kit comprises a squeezable and deformable container shaped like a bottle;
a cap attached to the container;
a first means of communication which permits a flow of liquid from the inside of the container towards the outside of the container and comprising a first passing duct made in the cap comprising a first fluid infeed section and a second fluid outfeed section;
a second means of communication which only permits the flow of air from the outside of the container towards the inside of the container;
a first draft tube with a fluid outfeed end section which can be removably attached to the first fluid infeed section of the first means of communication;
a dispenser comprising a duct having a first fluid infeed section and a second fluid outfeed end section;
a flexible connecting tube having a first fluid infeed section and a second fluid outfeed section where the first fluid infeed section of the connecting tube can be removably attached to the second fluid outfeed section of the first means of communication, and where the second fluid outfeed section of the connecting tube is attached to the first fluid infeed section of the third means of communication.

### Brief description of the drawings

Further characteristics and advantages of the invention will become apparent from the description of two preferred embodiments which follow with reference to the annexed drawings, given purely by way of a non-limiting examples, in which:
- Figure 1 is a diagram of the single components of the kit in a first embodiment of the present invention;
- Figures 2, 2A, 2B, 2C, 2D, 2E and 2F are respectively, a front view, a transparent front view showing the internal profiles, a cross-section along the line 2B-2B in Figure 2, a side view, a transparent side view showing the internal profiles, of a component of the first embodiment of the present invention;
- Figures 3 and 3A are perspective views of a component of the first embodiment of the present invention;
- Figures 4, 4A and 4B are respectively, a rear view, a cross-section along the line 4A-4A in Figure 4 and a detail view of part B in Figure 4A, of a first configuration of the first embodiment of the present invention;
- Figure 5 is a diagram showing a second configuration of the first embodiment of the present invention;
- Figure 6 is a diagram of the single components of the kit in a second embodiment of the present invention;
- Figures 7, 7A and 7B are respectively, a rear view, a cross-section along the line 7A-7A in Figure 7 and a detail view of part B in Figure 7A, of a first configuration of the second embodiment of the present invention;
- Figure 8 is a diagram showing a second configuration of the second embodiment of the present invention.

### Detailed description common to two embodiments of the invention

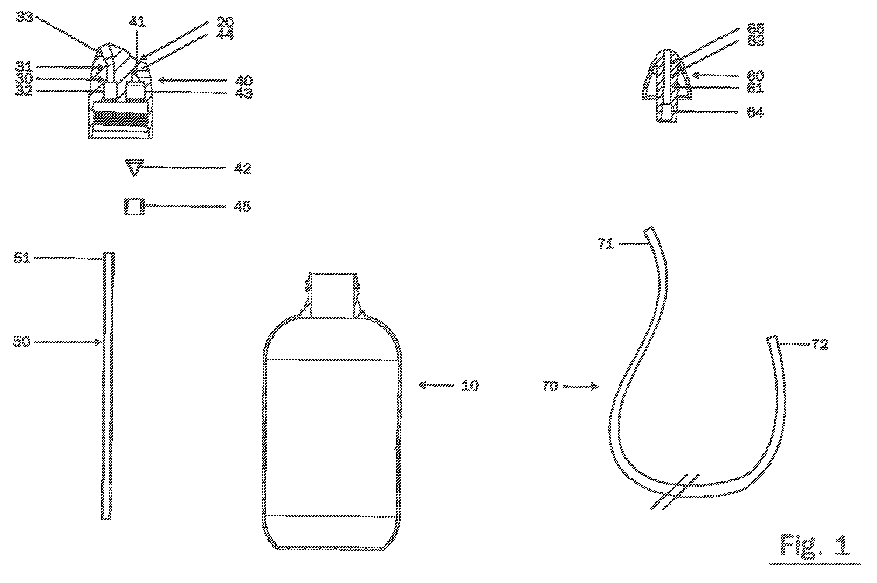

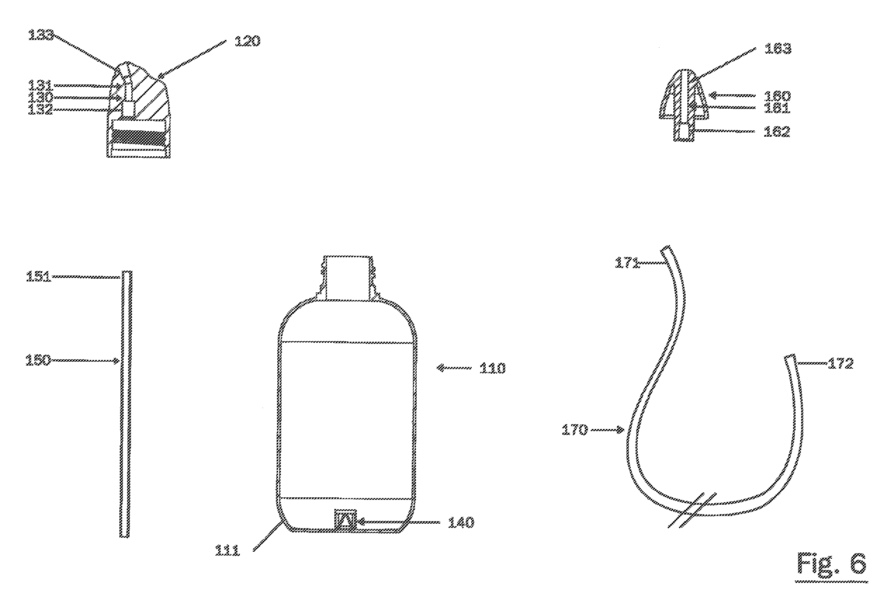

Figure 1 and the Figure 6 show respectively a first and a second embodiment of the present invention where the reference numbers common to both the first and the second embodiment are indicated together separated by a backslash ("/").

The kit according to the present invention comprises:
- a squeezable and deformable container 10/110 shaped like a bottle;
- a cap 20/120 attached to the container 10/110;
- a first means of communication 30/130 with a duct shape, where the means of communication 30/130 permit a flow of liquid from the inside of the container 10/110 towards the outside of the container 10/110 and where the first means of communication 30/130 comprise a first passing duct 31/131 in the cap 20/120, and where the first duct 31/131 comprises a first fluid infeed end section 32/132 and a second fluid outfeed end section 33/133;
- a second means of communication 40/140 which only permits the flow of air in one direction from the outside of the container 10/110 towards the inside of the container 10/110, and where a first draft tube 50/150 with a fluid outfeed section 51/151 which can be removably attached to the first fluid infeed end section 31/131 of the first means of communication 30/130;
- a dispenser 60/160 comprising a third means of communication 61/161 with a duct shape and having a first fluid infeed end section 62/162 and a second fluid outfeed end section 63/163;
- a flexible connecting tube 70/170 with a first fluid infeed section 71/171 and a second fluid outfeed section 72/172 where the first fluid infeed section 71/171 of the tube 70/170 can be removably attached to the second fluid outfeed section 33/133 of the first means of communication 30/130, and where the second section 72/172 of the tube 70/170 is attached to the first fluid infeed section 62/162 of the third means of communication 61/161.

### Detailed description of the first embodiment

Figure 1 shows the kit of components designed to form the first preferred embodiment of the present invention where the kit comprises
- a squeezable and deformable container 10 shaped like a bottle;
- a cap 20 attached to the container 10 having a first means of communication 30 with a first duct 31 and a second means of communication 40 with a second duct 41, and where the first duct 31 permits a flow from the inside of the container 10 towards the outside of the container 10, and where the second duct 41 only permits a flow from the outside of the container 10 towards the inside of the container 10, and where the first duct 31 comprises a first fluid infeed end section 32 and a second fluid outfeed end section 33;
- a first draft tube 50 with a fluid outfeed end section 51 which can be removably attached to the first fluid infeed section 32 of the first duct 31;
- a dispenser 60 having a duct 63 comprising a first fluid infeed section 64 and a second fluid outfeed section 65;
- a flexible connecting tube 70 with a first fluid infeed section 71 and a second fluid outfeed section 72 where the first fluid infeed section 71 of the connecting tube 70 can be removably attached to the second fluid outfeed section 33 of the duct 31, and where the second outfeed section 72 of the connecting tube 70 is attached to the first fluid outfeed section 64 of the third duct 63.

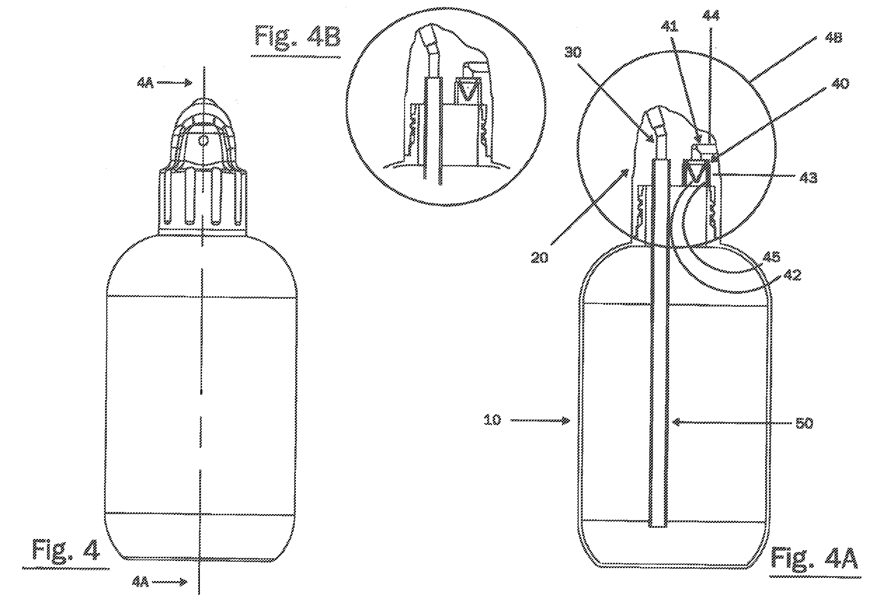

Figures 1, 4A and 4B show that the second means of communication 40 with a second duct 41 comprise a passing hole 41 made in the cap 20 and a one-way valve 42 located inside the passing hole 41.

The passing hole 41 comprises a first end section 43 which opens towards the inside of the cap 20 and communicates with the inside of the container 10 and a second end 44 which opens towards the inside and is located along the side wall of the cap 20.

In the embodiment described above, the end section 43 comprises a compartment which contains a one-way valve 42. In order to facilitate assembly, the valve 42 is held in position inside the compartment by a sleeve 45. Preferably these components are assembled with snap or interlocking fittings without the use of adhesives.

The one-way (non-return) valve 42 is preferably of the duckbill type but it should be noted that in this application any type of one-way valve or means with similar functioning could be used.

The second fluid outfeed end section 72 of the connecting tube 70 can preferably be removably attached to the first fluid infeed end section 64 of the third means of communication 61;

With the kit according to the present invention it is possible to configure the kit to perform nasal irrigation using at least two different methods as follows:
a) a first nasal irrigation method consisting of the squeezable method;
b) a second nasal irrigation method consisting of the application of drops.

Figures 4, 4A and 4B show the first configuration for performing the squeezable nasal irrigation method.

Only some of the components described above are used to form the first configuration. In other words, only a part of the components forming the kit are used for this configuration. Preferably the kit is marketed in a single package with the single components separated from each other.

Figures 4, 4A and 4B show the first squeezable configuration comprising the container 10, the cap 20 with the first (30) and second (40) means of communication and the draft tube 50. The user attaches the draft tube 50 by hand to the cap 20 by means of snap or interlocking fittings without the use of adhesives. The user screws the cap 20 to the neck of the container 10 where the screw fitting is preferably of the type without adhesive.

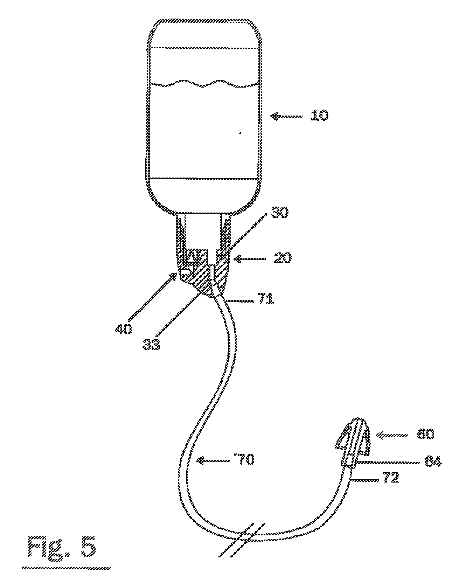

Figure 5 shows the second configuration for performing nasal irrigation by applying drops.

Only some of the components described above are used to form the second configuration. In other words, only a part of the components forming the kit are used for this configuration.

Figure 5 shows the second configuration for performing nasal irrigation by applying drops comprising the container 10, the cap 20 with the first (30) and second (40) means of communication, the flexible tube 70 and the dispenser 60.

The cap 20 is attached to the container 10 without the draft tube 50 preferably by means of screw fitting without the use of adhesive; the flexible connecting tube 70 is attached by hand by attaching its end section 71 to the outfeed section 33 of the first means of communication 30 and by attaching its opposite end section 72 to the infeed 64 to the dispenser 60, preferably using snap or interlocking fittings without the use of adhesive.

### Detailed description of the second embodiment

Figure 6 shows the kit of components designed to form the second preferred embodiment of the present invention.

This second embodiment comprises:
- a squeezable and deformable container 110 shaped like a bottle, comprising non-return communication means 140 which only permit the flow of air from the outside of the container 110 towards the inside of the container 110, and where the means 140 are preferably positioned close to the bottom 111 of the container 110;
- a cap 120 which can be attached to the container 110, comprising first means 130 consisting of a first duct 131 which permits the flow of liquid from the inside of the container 110 towards the outside and where the duct 131 comprises a first fluid infeed end section 132 and a second fluid outfeed end section 133;
- a first draft tube 150 with a fluid outfeed end section 151 which can be removably attached to the first fluid infeed end section 72 of the first communication means 130 forming a duct 131;
- a dispenser 160 comprising a third means of communication 161 with a duct shape and having a first fluid infeed end section 162 and a second fluid outfeed end section 163;
- a flexible connecting tube 170 with a first fluid infeed end section 171 and a second fluid outfeed end section 172 where the first fluid infeed section 171 of the tube 170 can be removably attached to the second fluid outfeed section 133 of the first means of communication 130, and where the second section 172 of the tube 170 is attached to the first fluid infeed section 162 of the third means of communication 161.

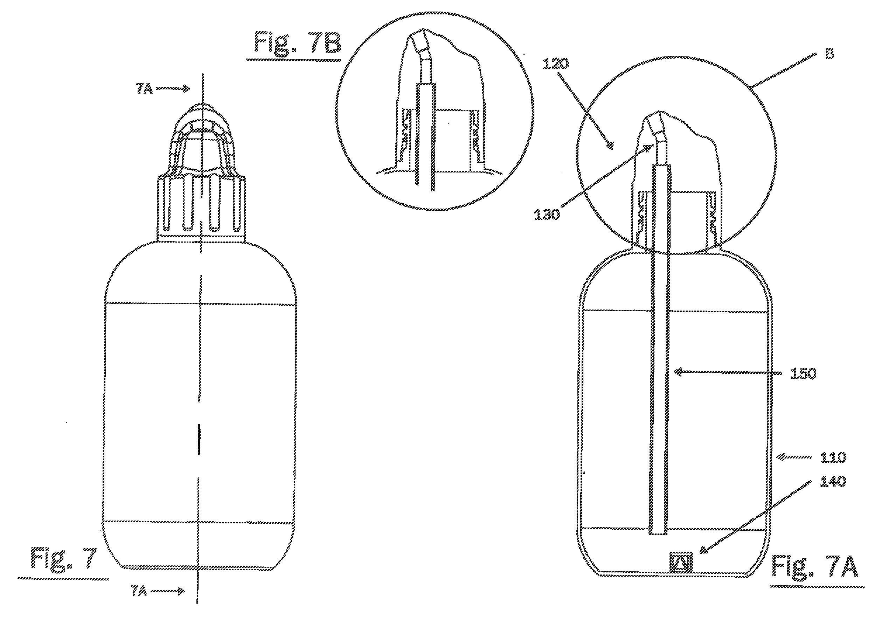

Figures 7, 7A and 7B show the first configuration for performing the squeezable nasal irrigation method.

Only some of the components described above are used to form the first configuration. In other words, only a part of the components forming the kit are used for this configuration.

Figures 7, 7A and 7B show the first squeezable configuration comprising the container 110 with its means of communication 140, the cap 120 with the means of communication 130 and the draft tube 150.

In the first configuration, the draft tube 150 is attached to the cap 120, preferably with snap or interlocking fittings without the use of adhesive and the cap 120 is attached to the container 110 preferably by means of a screw fitting without the use of adhesive.

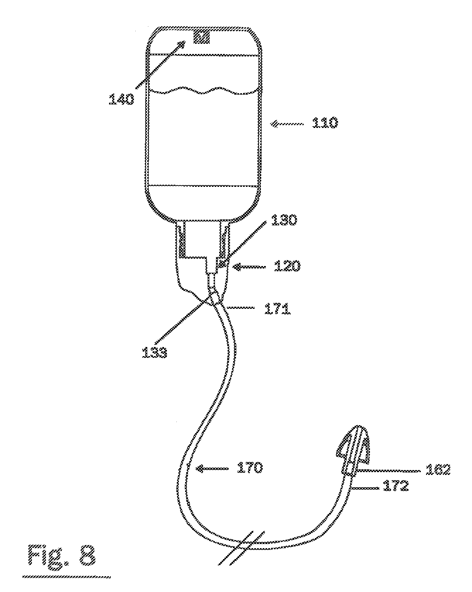

Figure 8 shows the second configuration for performing nasal irrigation by applying drops.

Only some of the components described above are used to form the second configuration. In other words, only a part of the components forming the kit are used for this configuration.

Figure 8 shows the second configuration for performing nasal irrigation by applying drops comprising the container 110 with the means of communication 140, the cap 120 with the means of communication 130, the flexible tube 170 and the dispenser 160.

In the second configuration, the cap 120 is attached to the container 110 without the draft tube 150 preferably by means of snap or interlocking fittings without the use of adhesive. The flexible connection tube 170 has an end section 171 attached to the outfeed section 133 of the first communication means 130 and the other end section 172 attached to the infeed section 162 of the communication means of the dispenser 160, preferably using snap or interlocking fittings without the use of adhesive.

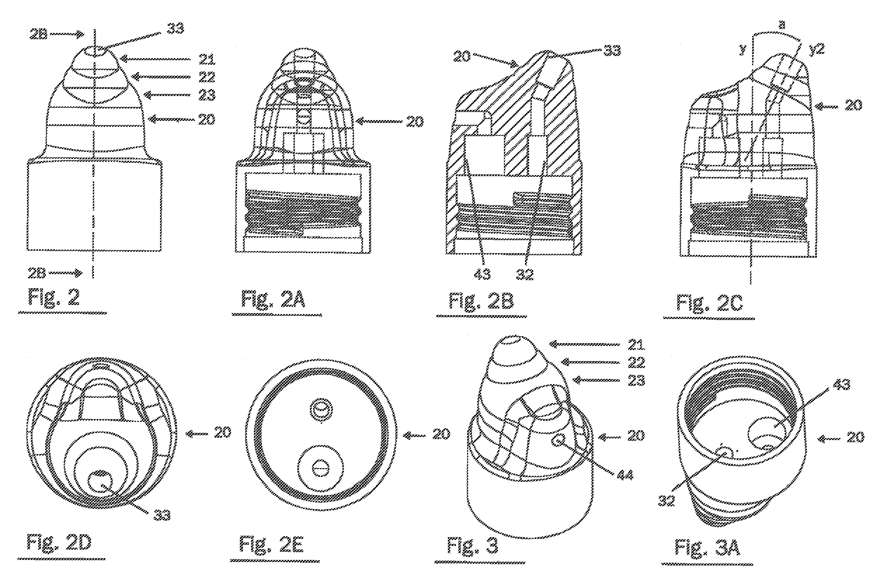

Figure 2C shows that preferably the cap 20/120 has a tapered tip angled to the side of the vertical axis Y of the cap 20/120 so as to form an angle a between the vertical axis Y and the geometrical axis Y2 of the tapered section.

Figure 2 shows that preferably the cap 20/120 has a tapered tip comprising two or more steps 21, 22, 23 where the steps increase in diameter from the tip to the base.

In particular, Figures 2 and 3 show that the cap 20/120 has a tapered tip comprising two or more circular steps 21, 22, 23 where the steps increase in diameter from the tip to the base and where the steps 21, 22, 23 are shaped to form a paraboloid segment.

## Claims

1. A kit for performing nasal irrigation by spraying or dropping a fluid into a nostril, comprising:
- a squeezable and deformable container (10/110) shaped like a bottle;
- a cap (20/120) attached to the container (10/110);
- a first means of communication (30/130) which permits a flow of liquid from the inside of the container (10/110) towards the outside of the container (10/110) and where the first means of communication (30/130) comprises a first duct (31/131) made in the cap (20/120) and where the first duct (31/121) has a first fluid infeed end section (32/132) and a second fluid outfeed end section (33/133);
- a second means of communication (40/140) which only permits the flow of air from the outside of the container (10/110) towards the inside of the container (10/110);
- a first draft tube (50/150) with a fluid outfeed end section (51/151) which can be removably attached to the first fluid infeed end section (32/132) of the first means of communication (30/130);
- a dispenser (60/160) comprising a third means of communication (61/161) with a duct shape and having a third fluid infeed end section (62/162) and a fourth fluid outfeed end section (63/163);
**characterised in that** said kit also comprises:
- a flexible connecting tube (70/170) with a first fluid infeed end section (71/171) and a second fluid outfeed end section (72/172) where the first fluid infeed section (71/171) of the connecting tube (70/170) can be removably attached to the second fluid outfeed end section (33/133) of the first means of communication (30/130), and where the second fluid outfeed end section (72/172) of the connecting tube (70/170) is attached to the first fluid infeed end section (62/162) of the third means of communication (61/161).

2. The kit according to claim 1, **characterised in that** the second means of communication (40/140) only permits a flow from the outside of the container (10/110) towards the inside of the container (10/110).

3. The kit according to claim 2, **characterised in that** the second means of communication (40) comprises a passing hole (41) made in the cap (20) and a one-way valve (42) located inside the passing hole (41).

4. The kit according to claim 3, **characterised in that** the one-way valve (42) is held in position by a sleeve (45).

5. The kit according to claim 2, **characterised in that** the second means of communication (140) comprises a one-way valve (140) located at the bottom of the container (110).

6. The kit according to one of the claims from 1 to 5, **characterised in that** it can be used in a first configuration designed to perform nasal irrigation by squeezing the container and can also be used in a second configuration designed to perform nasal irrigation by applying drops of fluid from the container.

## Patentansprüche

1. Kit zum Durchführen einer Nasenspülung durch Sprühen oder Tropfen eines Fluides in ein Nasenloch, welches Folgendes umfasst:
- einen quetschbaren und verformbaren Behälter (10/110), der wie eine Flasche geformt ist;
- eine Kappe (20/120), die an dem Behälter (10/110) angebracht ist;
- ein erstes Kommunikationsmittel (30/130), welches einen Flüssigkeitsstrom aus dem Inneren des Behälters (10/110) in Richtung des Äußeren des Behälters (10/110) gestattet, und wobei das erste Kommunikationsmittel (30/130) einen ersten Kanal (31/131), der in der Kappe (20/120) ausgebildet ist, umfasst, und wobei der erste Kanal (31/131) einen ersten Fluidzuführ-Endabschnitt (32/132) und einen zweiten Fluidabführ-Endabschnitt (33/133) aufweist;
- ein zweites Kommunikationsmittel (40/140), welches nur den Luftstrom von außerhalb des Behälters (10/110) in Richtung des Inneren des Behälters (10/110) gestattet;
- ein erstes Saugrohr (50/150) mit einem Fluidabführ-Endabschnitt (51/151), welches entfernbar an dem ersten Fluidzuführ-Endabschnitt (32/132) des ersten Kommunikationsmittels (30/130) angebracht sein kann;
- einen Dispenser (60/160), der ein drittes Kommunikationsmittel (61/161) mit einer Kanalform umfasst und einen dritten Fluidzuführ-Endabschnitt (62/162) und einen vierten Fluidabführ-Endabschnitt (63/163) aufweist;
**dadurch gekennzeichnet, dass** das Kit auch Folgendes umfasst:
- einen flexiblen Verbindungsschlauch (70/170) mit einem ersten Fluidzuführ-Endabschnitt (71/171) und einem zweiten Fluidabführ-Endabschnitt (72/172), wobei der erste Fluidzuführ-Endabschnitt (71/171) des Verbindungsschlauches (70/170) entfernbar an dem zweiten Fluidabführ-Endabschnitt (33/133) des ersten Kommunikationsmittels (30/130) angebracht sein kann, und wobei der zweite Fluidabführ-Endabschnitt (72/172) des Verbindungsschlauches (70/170) an dem ersten Fluidzuführ-Endabschnitt (62/162) des dritten Kommunikationsmittels (61/161) angebracht ist.

2. Kit nach Anspruch 1, **dadurch gekennzeichnet, dass** das zweite Kommunikationsmittel (40/140) nur einen Strom von außerhalb des Behälters (10/110) in Richtung des Inneren des Behälters (10/110) gestattet.

3. Kit nach Anspruch 2, **dadurch gekennzeichnet, dass** das zweite Kommunikationsmittel (40) ein Durchgangsloch (41), das in der Kappe (20) ausgebildet ist, und ein Einwegventil (42), das sich innerhalb des Durchgangsloches (41) befindet, umfasst.

4. Kit nach Anspruch 3, **dadurch gekennzeichnet, dass** das Einwegventil (42) durch eine Hülse (45) in Position gehalten wird.

5. Kit nach Anspruch 2, **dadurch gekennzeichnet, dass** das zweite Kommunikationsmittel (140) ein Einwegventil (140), das sich am Boden des Behälters (110) befindet, umfasst.

6. Kit nach einem der Ansprüche 1 bis 5, **dadurch gekennzeichnet, dass** es in einer ersten Konfiguration verwendet werden kann, die zum Durchführen einer Nasenspülung durch Quetschen des Behälters ausgelegt ist, und auch in einer zweiten Konfiguration verwendet werden kann, die zum Durchführen einer Nasenspülung durch Anwendung von Fluidtropfen aus dem Behälter ausgelegt ist.

## Revendications

1. Kit destiné à effectuer une irrigation nasale par pulvérisation ou application de gouttes d'un fluide dans une narine, comportant :
- un récipient compressible et déformable (10/110) en forme de bouteille ;
- un capuchon (20/120) fixé au récipient (10/110) ;
- un premier moyen de communication (30/130) qui permet un écoulement de liquide de l'intérieur du récipient (10/110) vers l'extérieur du récipient (10/110), le premier moyen de communication (30/130) comprenant un premier conduit (31/131) ménagé dans le capuchon (20/120), et le premier conduit (31/121) comporte une première section d'extrémité d'alimentation en fluide (32/132) et une seconde section d'extrémité de sortie de fluide (33/133) ;
- un deuxième moyen de communication (40/140) qui ne permet que l'écoulement d'air de l'extérieur du récipient (10/110) vers l'intérieur du récipient (10/110) ;
- un premier tube d'aspiration (50/150) pourvu d'une section d'extrémité de sortie de fluide (51/151) qui peut être fixé de manière amovible à la première section d'extrémité d'entrée de fluide (32/132) du premier moyen de communication (30/130) ;
- un distributeur (60/160) comprenant un troisième moyen de communication (61/161) ayant une forme de conduit et comportant une troisième section d'extrémité d'alimentation en fluide (62/162) et une quatrième section d'extrémité de sortie de fluide (63/163) ;
**caractérisé en ce que** ledit kit comprend en outre :
- un tube de raccordement flexible (70/170) pourvu d'une première section d'extrémité d'alimentation en fluide (71/171) et une seconde section d'extrémité de sortie de fluide (72/172), la première section d'entrée de fluide (71/171) du tube de raccordement (70/170) pouvant être fixée de façon amovible à la seconde section d'extrémité de sortie de fluide (33/133) des premiers moyens de communication (30/130), et la seconde section d'extrémité de sortie de fluide (72/172) du tube de raccordement (70 / 170) est fixée à la première section d'extrémité d'alimentation en fluide (62/162) du troisième moyen de communication (61/161).

2. Kit selon la revendication 1, **caractérisé en ce que** le deuxième moyen de communication (40/140) ne permet qu'un écoulement de l'extérieur du récipient (10/110) vers l'intérieur du récipient (10/110).

3. Kit selon la revendication 2, **caractérisé en ce que** le deuxième moyen de communication (40) comprend un trou de passage (41) ménagé dans le capuchon (20) et une valve unidirectionnelle (42) située à l'intérieur du trou de passage (41).

4. Kit selon la revendication 3, **caractérisé en ce que** la valve unidirectionnelle (42) est maintenue en position par un manchon (45).

5. Kit selon la revendication 2, **caractérisé en ce que** le deuxième moyen de communication (140) comprend une valve unidirectionnelle (140) située au fond du récipient (110).

6. Kit selon l'une des revendications 1 à 5, **caractérisé en ce qu'**il peut être utilisé dans une première configuration conçue pour effectuer une irrigation nasale par compression du récipient et peut également être utilisé dans une seconde configuration destinée à effectuer une irrigation nasale par appliquer de gouttes de fluide du récipient.
